# EUROPEAN PATENT SPECIFICATION

(11) **EP 1 429 822 B1**
(45) Date of publication and mention of the grant of the patent: **07.01.2009**
(21) Application number: 02720367.8
(22) Date of filing: 10.04.2002
(51) Int. Cl.: A61M 1/10

(54) **CARDIOPULMONARY LIFE SUPPORT SYSTEM**
KARDIOPULMONALES LEBENSERHALTUNGSSYSTEM
SYSTEME DE MAINTIEN DES FONCTIONS VITALES CARDIO-PULMONAIRES

(30) Priority: 25.09.2001 KR 2001059410; 07.12.2001 US 5537
(43) Date of publication of application: 23.06.2004
(73) Proprietor: Bio Heart & Kidney Inc., Gangnam-Gu Seoul 135-832 (KR)
(72) Inventor: MIN, Byoung G., Yongsan-gu, Seoul 140-133 (KR); WON, Yong, S., Dobong-gu Seoul 132-859 (KR); SUN, Kyung, Nowon-gu Seoul 139-841 (KR); BAEK, Kwang, J., Gangdong-gu Seoul 134-804 (KR); LEE, Hyuk, S., Seodaemun-gu Seoul 120-833 (KR); ROH, Yang, R., Gunsan-si Jeollabuk-do 753-280 (KR); HWANG, Chang, M., Jongno-gu Seoul 110-521 (KR); LEE, Jung, C., Dalseogu Daegu-si 704-927 (KR); LEE, Whang, J., Jowon-dong Jangan-gu Suwon-si Gyenggi-do 440-200 (KR)
(74) Representative: Mounteney, Simon James
(86) International application number: PCT/IB2002/001259
(87) International publication number: WO 2003/026723

(56) References cited:
- WO-A1-00/59560
- US-A- 3 046 903
- US-A- 3 720 485
- US-A- 3 842 440
- US-A- 5 033 943
- US-A- 5 300 111
- US-A- 5 577 891

## Description

### CLAIMING FOREIGN PRIORITY

The applicant claims and requests a foreign priority, through the Paris Convention for the Protection of Industry Property, based on a utility model application filed in the Republic of Korea (South Korea) with the filing date of September 25, 2001, with the utility model application number 10-2000-0032507, by the applicant.

### BACKGROUND OF THE INVENTION

The invention relates to an artificial heart for a patient requiring a cardiopulmonary life support in form of either artificial heart implantation or extracorporeal heart assistance. More specifically, the present invention relates to a cardiopulmonary life support system that substantially prevents blood clotting (thrombus) and dissolution or destruction of red blood cells (hemolysis) from occurring in blood vessels of a heart patient who receives its assistance.

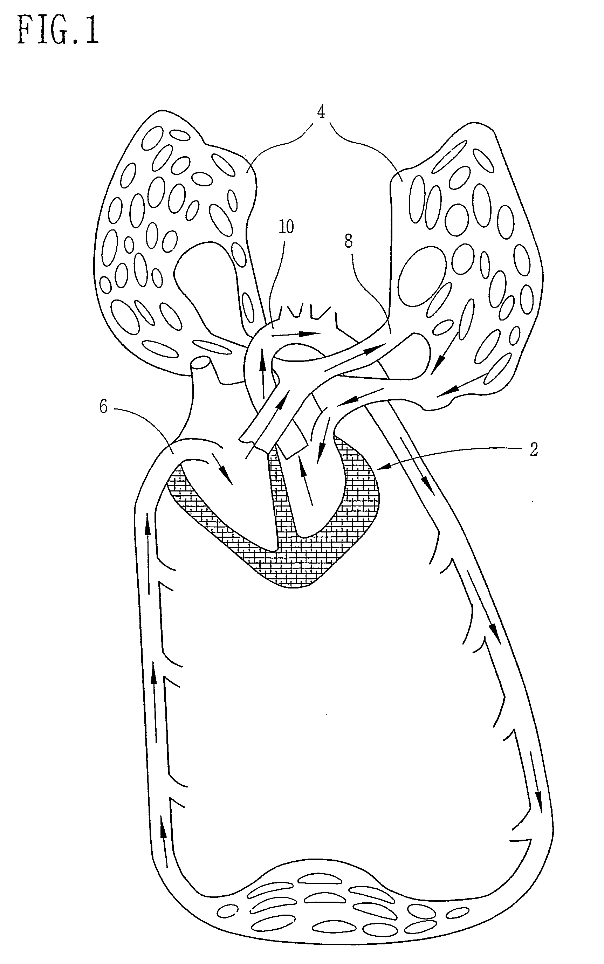

FIG. 1 is a schematic view showing a heart **2**, lungs **4** and a blood circulation in a mammal or a human, wherein arrows indicate direction of the blood circulation. As shown therein, the heart **2** includes two atriums above and two ventricles below. A main vein **6** is connected to the right atrium and the right ventricle is linked to a pulmonary artery **8**. The lungs are connected to the left atrium and the left ventricle is linked to an aorta **10**. Regular pumping of the left ventricle pushes out blood therein into the aorta **10** to deliver nutrition and oxygen to each capillary vessel in the body. Meanwhile, the blood with less oxygen is in turn collected in the main vein that links to the right atrium to complete a blood circulation known as a systematic circulation. The oxygen-depleted blood collected in the right atrium is released down to the right ventricle and sent to each lung through the pulmonary artery for blood oxygenation. The blood oxygenated in the lungs is released through the left atrium down to the left ventricle. Through the blood circulation for blood oxygenation also known as a pulmonary circulation, the oxygen-depleted blood is converted to an oxygen-rich blood and collected back in the left ventricle. The oxygen-rich blood collected in the left ventricle repeats the systematic circulation in accordance with the regular pumping which generates rhythmic pulses. A valve in each atrium and ventricle serves to prevent a reverse stream.

Each rhythmic pulse in the atriums and ventricles differs depending on age, sex and physical condition. However, the heart pulse frequency for an individual is regular in a stabilized condition. A standard per-minute heart pulse frequency is known to range about 100 to 140 for infants, 80 to 90 for elementary school kids, 60 to 80 for young and middle aged adults, and 60 to 70 for senior people. Male tends to be less in pulse frequency than female. In general, the smaller the body, the more frequent becomes the heart pulsation for animals. If the body-surface area is larger than the body volume, heat emission becomes further invigorated and thus blood circulation should be faster to complement the loss resulting from the heat emission. For example, the per-minute pulse frequency ranges about 30 to 40 for elephants, 90 to 90 for dogs, 140 to 160 for rabbits, and 200 to 300 for rats. The pulse frequency in an artificial heart can be adjusted by controlling the rotation of a motor that drives the artificial heart.

The heart along with lungs is the most crucial organ that allows a living body to maintain its life. However, the heart should remain motionless and emptied in order to conduct a precise surgical heart operation. Therefore, considering the vitality of the heart without which the life does not last more than five minutes, an artificial heart or cardiopulmonary assistance device should be inevitably utilized in such life threatening urgent circumstances as a heart attack, a sepsis related shock, or a myocardium infraction.

Many studies on artificial hearts have been focused on blood pumping which most affects functioning of an artificial heart in a body. The leading conventional arts regarding artificial hearts will be now briefly described focusing each function of blood pumping.

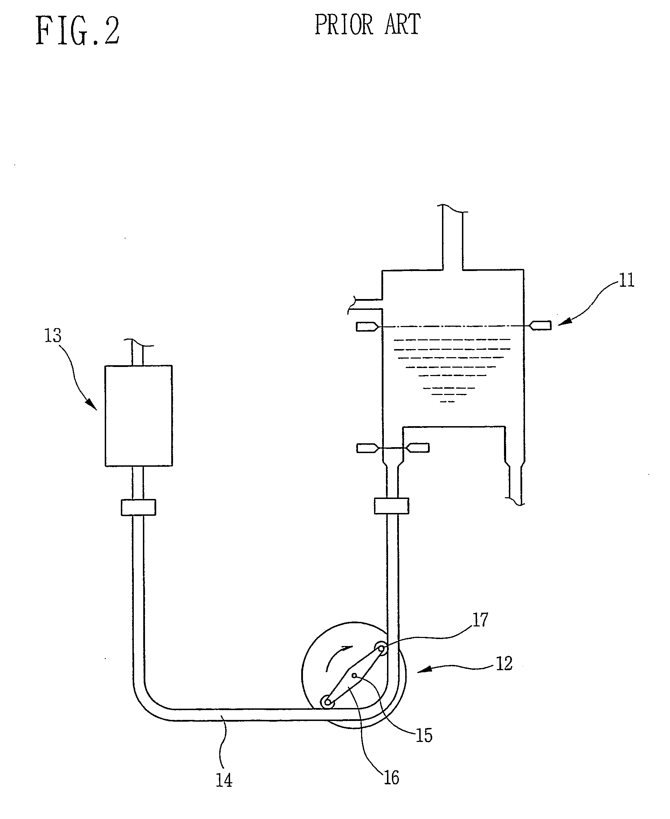

FIG. 2 is a view showing a conventional cardiopulmonary device using a rotary pump. As shown therein the rotary pumping device includes a blood storage, a rotary type pump **12**, an oxygenator **13**, and a flexible tube **14**. The blood storage **11** stores therein a blood from a main vein of a patient. The rotary blood pump **12** serves to transfer the blood from the storage **11** to the oxygenator **13**. The flexible tube **14** links the blood storage **12** and the oxygenator **13**. The flexible blood tube **14** is arc-bent by 90 degrees around the rotary blood pump **12**. A rotation shaft **15** is radially formed from the arc-bent portion of the tube **14** through the center of the rotary pump **12**. A rotation arm **16** is engaged to the rotation shaft **15** and two rotary rollers **17** are rotatably provided to rotate in accordance with the rotation shaft **15**. The rotation of the shaft **15** allows the pump **12** to serve to make a sequential squeezing rotation along the arc-bent portion of the tube **14**. However, the squeezing rotation of the pump **12** fails to generate a stable, pulsatile blood pumping. Further, the excessive pressure for the squeezing rotation tends to easily lead to thrombosis and hemolysis in the oxygenator **13**. Also, the rotary pump **12** is only usable for about 6 to 8 hours which substantially limits its application to a time taking surgical heart operation.

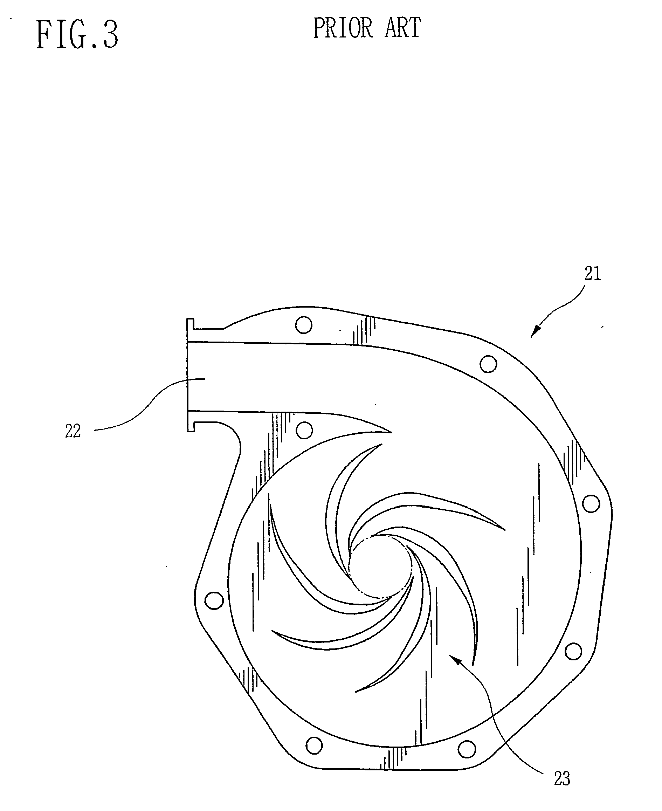

FIG. 3 shows a schematic cross-sectional view of a conventional centrifugal blood pump **21**. The centrifugal blood pump **21** includes an input port (not shown) to receive blood from a flexible tube (not shown) connected to a right atrium, an output port **22** to release the blood from the blood pump **21**, and an impeller **23** having blades. The rotation speed of the impeller **23** can be adjusted depending on a patient. However, since the blood in the centrifugal blood pump **21** becomes in contact with either the inner surface of the blood bump **21** or mechanical surfaces of the impeller **23**, there may easily occur blood clotting or blood dissolution.

In particular, the damage incurrence on red blood cells or blood platelets due to the blood clotting and dissolution is determined by stress resulting from the blood flow in the pump **21** and by how long the blood has stayed in the pump **21**. Also, the stress due to the blood flow is determined by the rotation speed of the impeller **23** and by the asperity of the mechanical surfaces, thereby increasing possibility of blood damage. The time period in which the blood stays in the centrifugal blood pump **21** is a major factor to consider in the pump design. A shear stress sufficient to affect the blood staying in the pump may lead to thrombosis resulting from congelation, embolism or fibrin accumulation on the inner surface of the pump. There may also occur blood dissolution or red cell destruction due to a flow separation, a cavitation, or a solution swirl which may be caused by the rotation of the impeller **22**. Therefore, the centrifugal blood pump **21** can be utilized for a limited time period like the rotary blood pump.

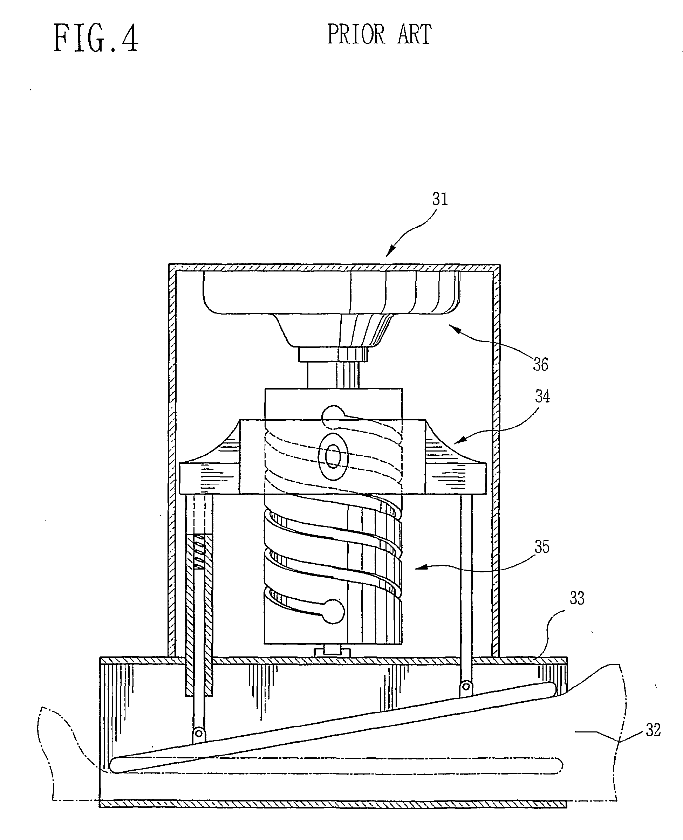

FIG. 4 shows a conventional pulsatile blood pump **31**. As shown therein, the pulsatile pump **31** includes a bag tube **32**, a pressure plate **33**, a plate support **34**, a rotation body **35**, and a drive motor **36**. The bag tube **32** is provided with a valve (not shown) at each end thereof. The pressure plate **33** pressurizes the tube **32** for blood transfer. The plate support **34** supports and vertically shuttles the pressure plate **33**. The rotation body **35** is threaded to allow the plate support **34** to make a vertical reciprocal movement.

When the pressure plate **33** the plate support **34** are lowered according to the rotation body **35** driven by the motor **36**, the blood is discharged from the tube **32**, and when raised the blood is supplied into the tube **32**, thereby enabling the pulsatile blood pumping. However, the pulsatile blood pump **31** may cause friction by the contact of the rotation body and the plate support **34** to thereby undermine a stabilized reciprocal movement. Further, the reciprocal rotation of the drive motor **36** that drives the rotation body **35** may increase pressure for pumping the blood to the oxygenator, thereby incurring thrombosis and hemolysis.

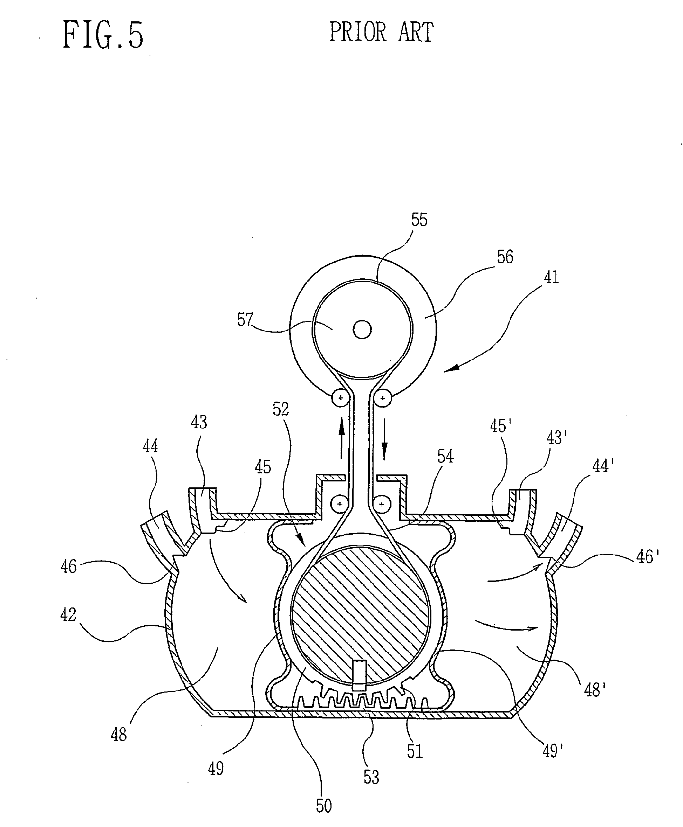

FIG. 5 shows a conventional dual pulsatile blood pump **41**. As shown therein, the pulsatile pump **41** includes input ports **43**, **43**', output ports **44**, **44**', input valves **45**, **45**', and output valves **46**, **46**'. Each valve is formed in a corresponding one of the ports. The pump **41** also includes a pump case **42** that houses therein a spherical body **52**. The spherical body **52** has a groove **50** therearound and a gear **51**. The gear **51** is engaged to a rack **53** attached to an inner wall of the pump case **42**. A rubber membrane **49**, **49**' covers the gear **51**, rack **53** and the groove **50**. A belt **54** is carried in along the groove **50** of the body **52** and around a pulley **57** linked to a motor **56**. A tension applied to the pulley **57** together with the engagement of the gear **51** and the rack **53** enables a shuttling movement of the spherical body **52**, whereby the body **52** makes a horizontal shuttle movement to pump the blood in the blood chamber **48**. The dual pulsatile blood pump **31** substantially decreases thrombosis and hemolysis compared to the rotary pump or other pulsatile pumps. However, the mechanical surfaces are exposed to the blood except for the rubber membranes **49, 49**' and the input and output ports are also exposed to mechanical surfaces, which may still incur thrombosis and hemolysis. Further, the streamline formation around the input and output ports in the pump chamber **48**, **48**' may lead to pressure loss which easily results in blood clotting or blood dissolution. In addition, the continued friction and stress may serve to elongate the belt and this makes it difficult to maintain stable pulsation and blood pressure. Also, the conventional dual pulsatile blood pump 41 substantially increases production cost due to mechanical requirements for the shuttle movement of the spherical body 41.

WO 00-59560 relates to an implantable ventricular assist device that is used to replace the function of the right ventricle, the left ventricle or both ventricles of the heart. US 5577891 describes a resuscitation pump and a method for pumping liquid or fluid through a pair of resilient tubes including a pulsing mechanism which partially compresses the tubes in an alternating manner. US 5033943 describes a pump for pumping liquids used in biochemical procedures. US 3046903 describes an artificial blood circulation apparatus.

### SUMMARY OF INVENTION

The invention is contrived to overcome the conventional disadvantages. Accordingly, an object of the present invention is to provide a cardiopulmonary life support system that substantially prevents blood clotting (thrombosis) and dissolution or destruction of red blood cells (hemolysis) from occurring in blood vessels of a heart patient who receives its assistance.

Another object of the invention is to enable a heart patient to use the life support system for a longer time period in form of either extracorporeal life support or surgical implantation. A further object is to improve portability for an extracorporeal system application and to minimize the size of the life support system to facilitate implantation. A still further object is to realize a rhythmic pulsation substantially equivalent to the systematic pulsation in a living body.

According to the present invention there is provided a cardiopulmonary life support system as set out in Claim 1. Preferred features are set out in Claims 2 to 22.

The advantages of cardiopulmonary life support system according to the present invention are numerous. Initially, the gently alternating reciprocal movement of the alternating member squeezes the first and second tubes sequentially, alternately, gently and efficiently for blood pumping operation so that the oxgenator becomes less pressurized by the repeated blood pumping, thereby substantially decreasing incurrence of blood clotting (thrombosis) and dissolution or destruction of red blood cells (hemolysis), which are known as common side effects to most patients receiving assistance of conventional artificial hearts.

Further, the first and second tubes are formed of a flexible, resilient material and the solid alternating member is operatively provided between the first and second tubes in such a simplified, stabilized construction that the expected life span of the life support system is substantially extended without system replacement. In addition, the alternating member and the first and second tubes are efficiently accommodated within the housing to alternately enable each blood pumping operation for the first and second tube in such a limited space that a significant system size decrease is realized, for example, from a conventional refrigerator size to a palm size in an implantation version of the present invention or to a portable size in an extracorporeal assistance version of the present invention.

Also, the gentle, pulsatile blood pumping operation accomplished within the housing in systematic combination of the flexible blood tubes and the gently alternating solid member generates safe and steady blood pulses substantially similar to those of a natural heart, thereby improving product reliability. More importantly, the artificial blood pumping system adapting the alternately tube-squeezing mechanism requires less elements and further simplifies the overall structure for the blood pumping operation, thereby substantially decreasing production cost, whereby a surgical implantation of the life support system may be realized, for example, within about one and half times the medical bill charged for a large surgical heart operation.

Although the present invention is briefly summarized, the fuller understanding of the invention can be obtained by the following drawings, detailed description and appended claims.

### BRIEF DESCIPTION OF THE DRAWINGS

These and other features, aspects and advantages of the present invention will become better understood with reference to the accompanying drawings, wherein:
FIG. 1 is a schematic view showing a heart, lungs and a blood circulation in a mammal or a human;
FIG. 2 is a structural view showing a conventional cardiopulmonary device using a rotary blood pump;
FIG. 3 is a schematic cross-sectional view of another conventional cardiopulmonary device using a centrifugal blood pump;
FIG. 4 is a structural view showing a further conventional cardiopulmonary device using a pulsatile blood pump;
FIG. 5 is a cross-sectional view showing a still further conventional cardiopulmonary device using a dual pulsatile blood pump;
FIG. 6 is a perspective view showing a cardiopulmonary life support system according an embodiment of the present invention;
FIG. 7 is a perspective view detailing a blood pump unit in FIG. 6;
FIG. 8 is a cross-sectional front view of the blood pump unit in FIG. 6;
FIG. 9 is a cross-sectional top view of the blood pump unit in FIG. 6;
FIG. 10 is a cross-sectional side view of the blood pump unit in FIG. 6;
FIG. 11 is a schematic structural view of a pump drive unit in FIG. 6;
FIG. 12 is a top view of a link unit in FIG. 11;
FIG. 13 is a cross-sectional view of a male spline in FIG. 6;
FIG. 14 is a cross-sectional front view of the blood pump unit in FIG. 6 according to another embodiment of the present invention;
FIG. 15 is a structural view of a blood pump unit according to another embodiment of the present invention; and
FIG. 16 is a cross-sectional top view of the blood pump unit in FIG. 15.

### DETAILED DESCRIPTION OF THE PREFERRED EMBODIMENTS

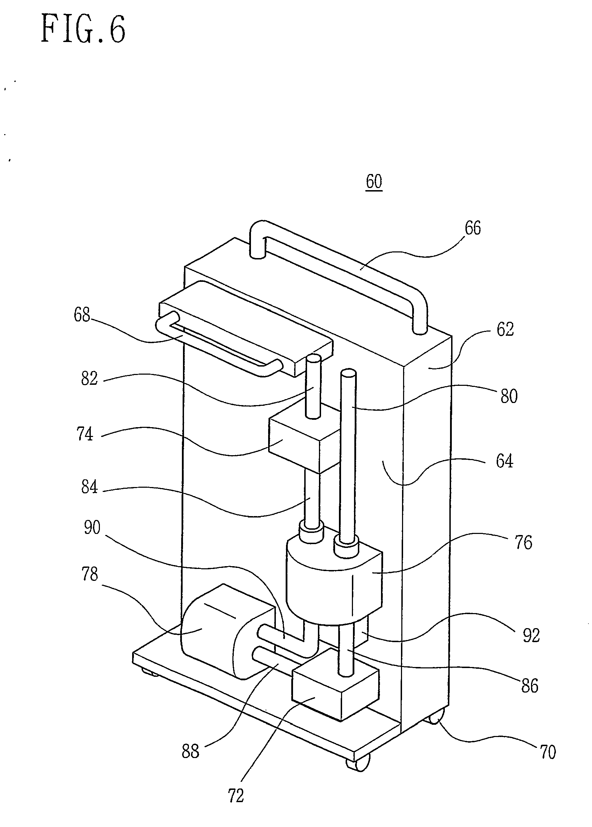

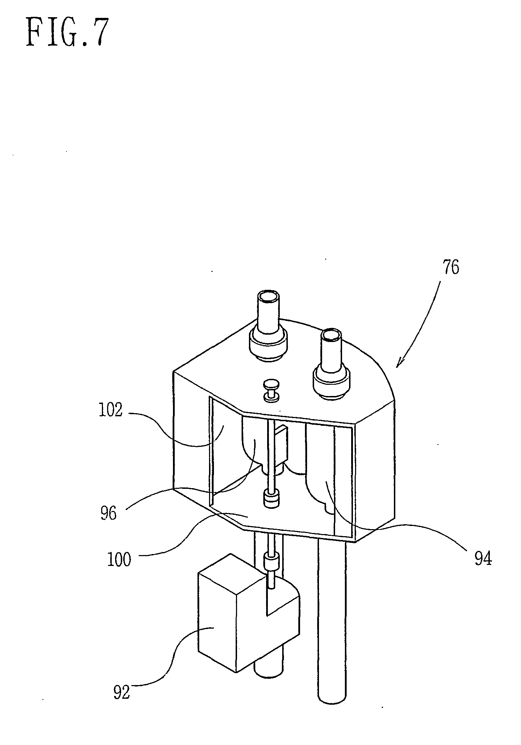

As shown in FIGS. 6 and 7, a cardiopulmonary life support system **60** according to an embodiment of present invention includes a support body **62** having a side wall **64**. The support body **62** is selectively provided with handles **66**, **68** and rollers **70** to facilitate portability and to improve mobility of the system 60. On the side wall **64** are attached first and second blood storages **72, 74**, a blood pump housing **76**, and an oxygenator **78**, which are linked by ducts **80**, **82**, **84**, **86**, **88**, **90**. In this construction, a general blood stream is sequentially made through the duct **82** to be connected to a main vein of a patient, the second storage **74**, the duct **84**, the second tube **96** in the housing **76**, the duct **90**, the oxygenator **78**, the duct **88**, the first blood storage **72**, the duct **86**, the first tube **94** in the housing **94**, and the duct **80** to be connected to an aorta of the patient. Here, each duct is formed of a flexible material to facilitate blood flowing therethrough.

To see a general arrangement of each element for the life support system **60**, the first blood storage **72** is formed between the oxygenator **78** and the first tube **94** via the ducts **88**, **86** to temporarily store therein an oxygen-rich blood oxygenated in the oxygenator **78**. The second blood storage **74** is connected to the second tube **96** via the duct **84**. The second storage **74** temporarily stores therein an oxygen-depleted blood that flows therein from the main vein via the duct **82**.

When the life support system **60** is applied to a patient requiring an extracorporeal heart assistance, an oxygen-depleted blood flows in through the duct **82** that is to be connected to a main vein of the patient and is oxygenated in the oxygenator **78**. In accordance with a pumping operation in the housing **76,** the oxygenated, oxygen-rich blood flows out through the duct **80** that is to be connected to an aorta of the patient. The pumping operation in the housing **76** is driven by a motor unit **92** that is adjacent to the housing 76. The housing **76** is defined by a top side **98**, a bottom side **100**, and an inner periphery **102**.

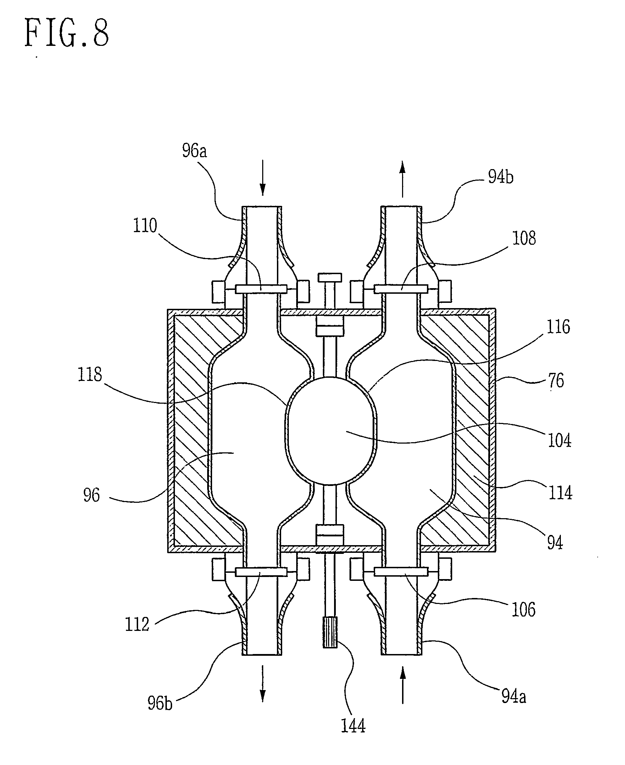

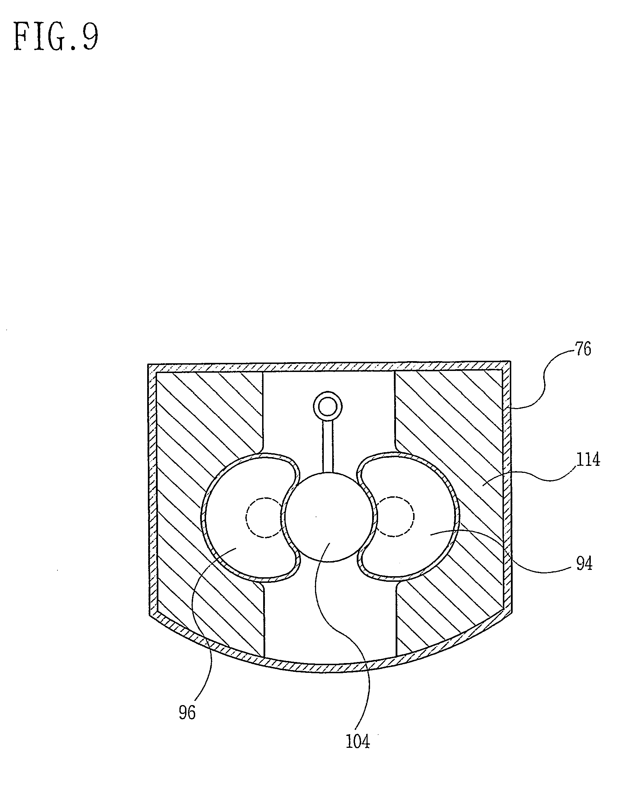

FIGS. 8, 9 and 10 specify construction of a blood pumping mechanism of the life support system **60**. As shown therein, the first and second tubes **94**, **96** are provided adjacent to each other in the housing **76**. The first and second tubes **94**, **96** each have an input port **94a**, **96a** and an output port **94b**, **96b**. An alternating member **104** is disposed between the tubes **94**, **96** and attached to the housing **76** so as to alternately squeeze the first and second tubes **94**, **96**. That is, the alternating member **104** serves to partially thrust each tube **94**, **96** in a sequential or an alternating order in accordance with the motor unit **92** to efficiently control a blood flow in and out of the first and second tubes **94**, **96**. Preferably, the alternating member **104** is solid in a spherical shape. The alternating member **104** may be also shaped in a capsule type with a solid formation. The first and second tubes **94**, **96** are linearly aligned and substantially parallel with each other in the housing **78**.

The life support system 60 further comprises valves **106, 108**, **110**, **112** each of which is a one-way valve to allow a single directional stream therethrough. The valves **106**, **108**, **110**, **112** are sequentially formed in the input and output ports **94a**, **94b**, **96a**, **96b** to prevent a reverse stream in the first and second tubes **94**, **96**. So the blood flows into the first tube **94** through the first input port **94a** in which the first input valve **106** that is a one-way valve disposed in the first input port **94a** blocks the reverse stream from the first tube **94**. Also, the blood in the first tube **94** may flow out through the first output port **94b** in which the first output valve **108** that is a one-way valve blocks a possible reverse stream from the duct **80** into the first tube **94**. Likewise, the second input valve **110** in the second input port 96a prevents a reverse stream from the second tube **96** and the second output valve **112** in the second output port 96b serves to prevent a reverse stream from the duct **90** linked to the oxygenator **78** into the second tube **96**.

In order to improve efficiency of the sequential pumping operation in the housing **76**, the life support system **60** also includes a tube support **114** that is fitted between each tube **94**, **96** and the inner periphery **102** of the housing **76**. The tube support **114** is formed in a solid material and substantially spaced from the alternating member **104.** At least, each tube portion **116, 118** that makes direct contacts with the alternating member **104** becomes protected from the tube support **114.** Namely, the solid tube support **114** does not in the least prevent activation of the alternating member **104** but serves to stabilize the pumping operation in the housing **76** despite the flexible characteristic of each tube **94, 96.** Selectively, the tube support 114 may be formed of either a substantially solid material or a substantially rigid material.

The first and second tubes **94, 96** are each formed of a flexible material. Further, each tube **94, 96** may be formed of a polymer known to well harmonize with a mammal body in terms of either a surgical operation or a bodily implantation. Selectively, the first and second tubes **94, 96** may be formed of silicon. According to such a material characteristic, the first and second tubes **94, 96** are each elastically, substantially restored to its original shape after being squeezed by the alternating member **104.**

To improve usability of the life support system **60,** the input port **96a** for the second tube **96** and the output port **94b** for the first tube **94** are each formed through the top side **98** of the housing **76.** That is, the output port **94b** of the first tube **94** passes through the top side **98** and the output port **96b** of the second tube **96** passed through the bottom side **100** of the housing **76**.

Importantly, the alternating member **104** serves to generate an artificial rhythmic pulsation substantially similar to the natural blood pumping in the heart of a living body. Specifically, an initial squeezing of the alternating member **104** on the first tube **94** enables a blood to partially pump out from the first tube **94** through the first tube output port **94b.** A subsequent squeezing of the alternating member **104** on the second tube **96** enables the blood to partially pump out from the second tube **96** through the second tube output port **96b** while a restoration of the first tube **94** to its original shape enables the first tube **94** to suck in as much as pumped out therefrom through the first input port valve **106**. And, a further subsequent squeezing of the alternating member **104** on the first tube **94** enables the blood to partially pump out from the first tube **94** through the first tube output port **94b** while a subsequent restoration of the second tube **96** to its original shaft enables the second tube **96** to suck in as much as pumped out therefrom through the second input port valve **110**.

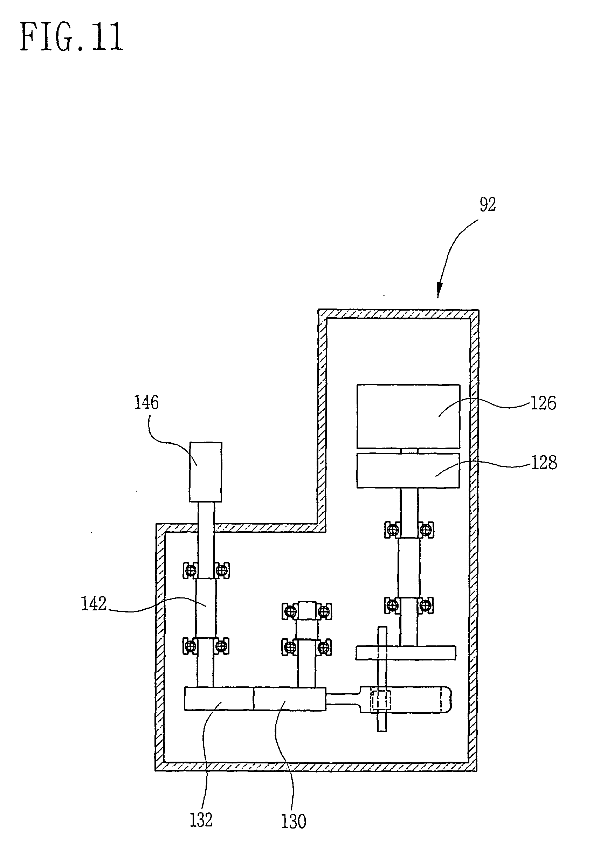

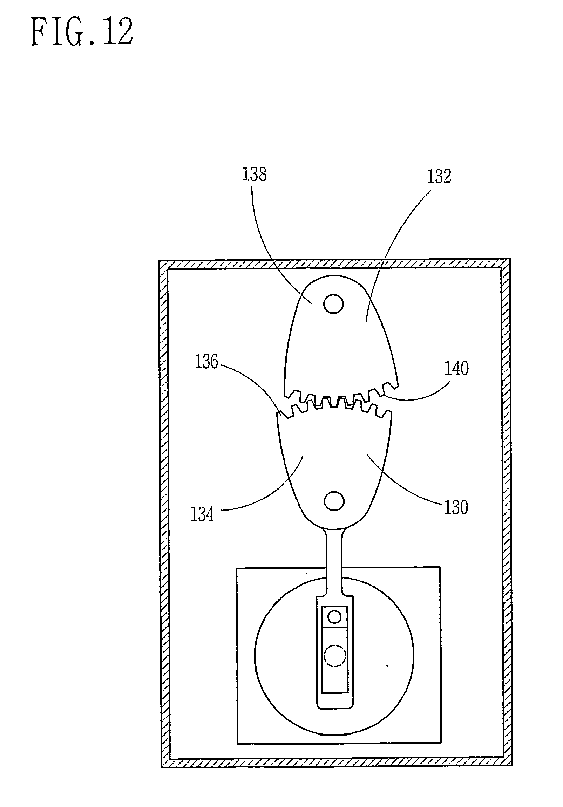

FIGS. **11**, **12** and **13** each show a mechanism to actuate the alternating member **104**. As shown therein, the life support system **60** further includes a shaft **116** having a top portion **118**, a mid portion **120**, and a bottom portion **122**. The shaft 118 is connected to the motor unit **92** and substantially parallel to the tubes **94**, **96**. The top portion **118** of the shaft **116** is rotatably attached to the top side **98**, the mid portion **120** is fixedly attached to the alternating member **104**, and the bottom portion **122** rotatably passes through the bottom side **100** of the housing **76**, whereby an angular reciprocal rotation of the shaft **116** enables the alternating member **104** to alternately squeeze the first and second tubes **94**, **96**.

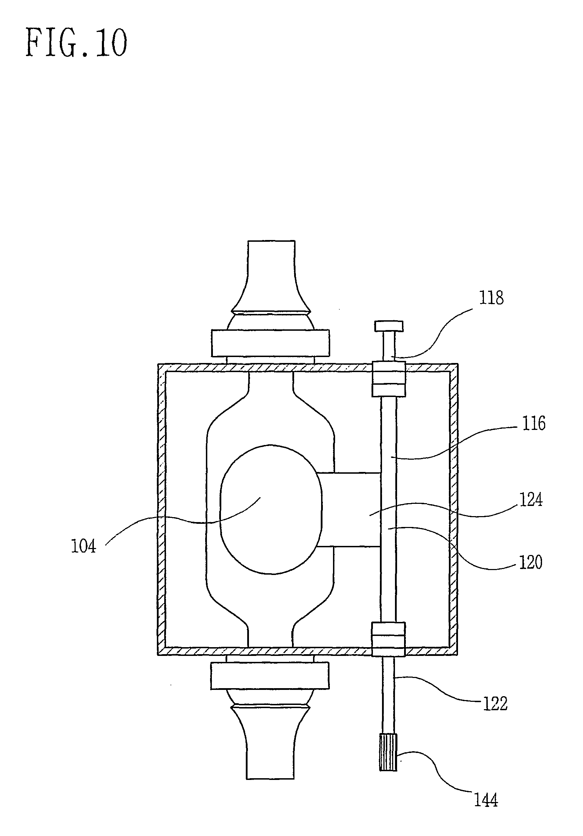

As shown back in FIG. **10**, a support plate **124** is formed between the alternating member **104** and the mid portion **120** of the shaft **116**. That is, the support plate **124** extends from the mid portion **120** of the shaft **116** fixedly to the alternating member **104**. In order to actuate the shaft **116**, the motor unit **92** includes a motor **126**, a decelerator **128**, first and second gears **130**, **132**. The decelerator **126** is connected to the motor **126** to moderate a torque from the motor **126**. The first gear **130** has a base **134** and gear teeth **136**, and the second gear **132** has a base **138** and gear teeth **140**. The first gear base **134** is connected to the decelerator **128**, and the first gear teeth **136** is rotatably connected to the second gear teeth **140** of the second gear **132**. A connecting rod **142** extends from the second gear base **138**.

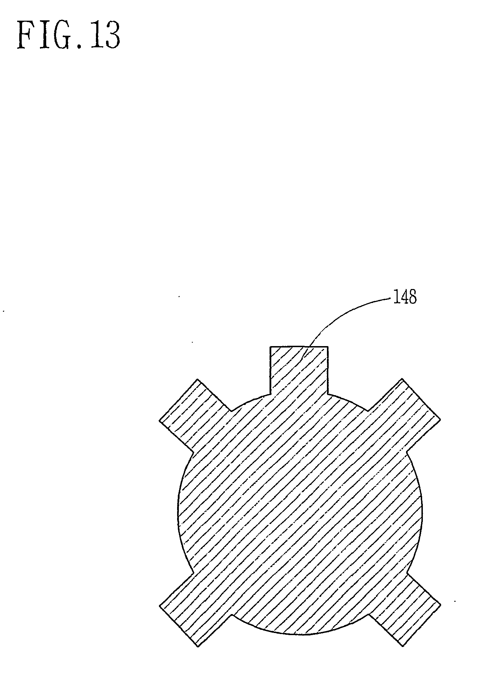

Meanwhile, male and female splines **144**, **146** are detachably provided between the shaft **116** and the connecting rod **142**. That is, the male spline **144** in FIG. **8** is attached to the bottom portion **122** of the shaft **116** and detachably engaged to the female spline **146**, in FIG. **11**. The female spline **146** is linked to the second gear base **132** via the connecting rod **142**. So the rotational torque generated by the motor **126** and moderated by the decelerator **128** is converted to an angular reciprocal rotation in accordance with the first and second gears **130**, **132**. Specifically, the first and second gear teeth **136**, **140** are each formed in an arc rack to enable generation of the angular reciprocal rotation. For a reliably detachable engagement between the male and female splines **144**, **146**, the matching teeth may be shaped in a safety formation as shown in FIG. **13**. It is recommended that teeth **148** in each spline **144**, **146** be formed in an irregular alignment.

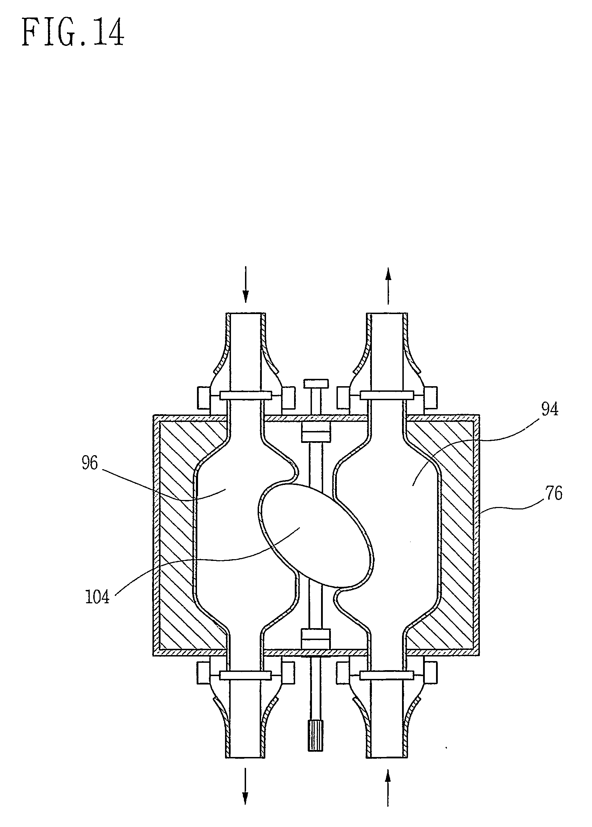

The alternating movement of the alternating member **104** is preferably implemented in a horizontal direction between the first and second tubes **94**, **96**. The alternating member **104** may be provided in a slanting ellipsoid to realize an alternate diagonal squeezing on the first and second tubes **94**, **96** as shown in FIG. **14**.

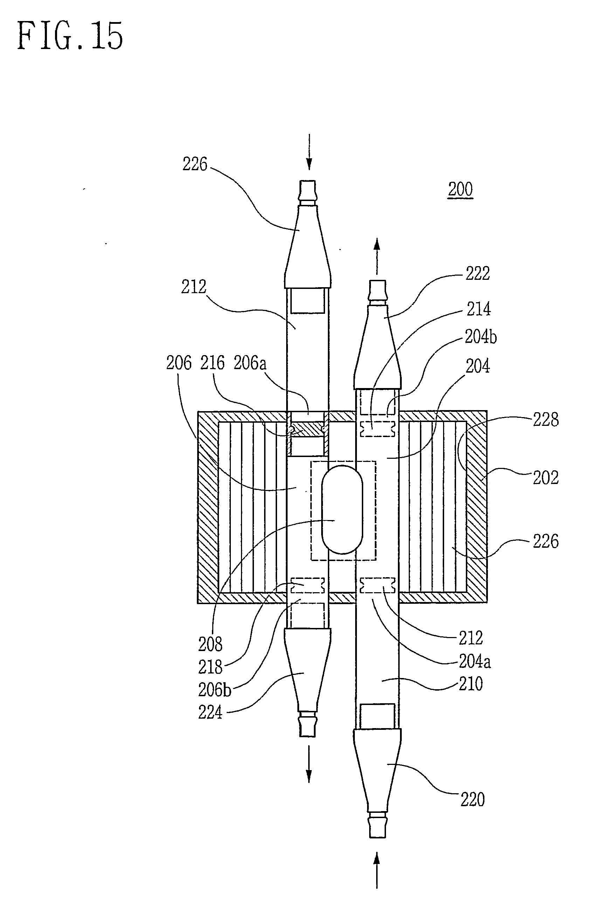

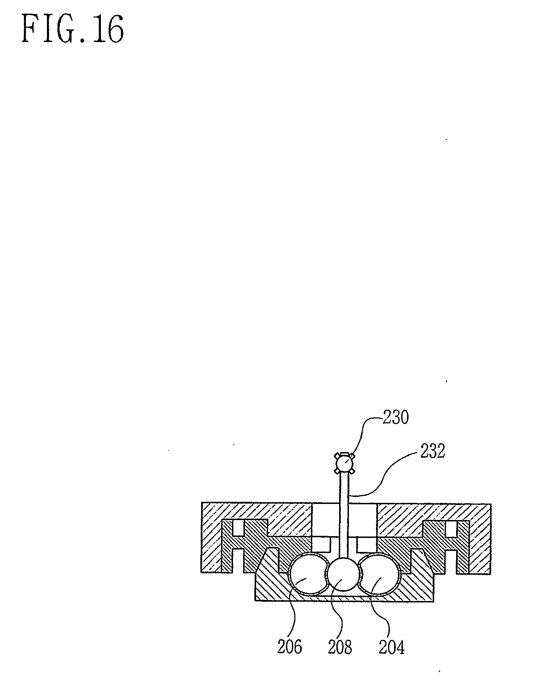

In FIGS. **15** and **16**, a cardiopulmonary life support system **200** according to another embodiment of the present invention is provided in a decreased formation in size to fit for its surgical implantation. As shown therein, the life support system **200** includes a housing **202**, first and second tubes **204**, **206**, an alternating member **208**, a first blood storage **210**, and a second blood storage **212**. Each tube **204**, **206** has input and output ports **204a**, **204b**, **206a**, **206b** each of which sequentially includes valves **212**, **214**, **216**, **218** therein to allow a one-way stream in and out of the tubes **204**, **206**.

Also, the first blood storage **210** serves to temporarily store therein an oxygenated blood. The second blood storage **212** temporarily reserves therein an oxygen-depleted blood. Short ducts **220**, **222**, **224**, **226** may be provided depending on requirements. A solid tube support **226** is formed between each tube **204**, **206** and an inner periphery **228** of the housing **202** to stabilize the blood pumping operation in accordance with the alternating member **208** which is attached to a shaft **230** via an extension **232**.

In this construction, the angular reciprocal rotation of the shaft **230** enables the alternating member **208** to alternately make a horizontal squeezing on the first and second tubes **204**, **206.** Accordingly, the first tube **204** regularly pumps out the oxygen-rich blood through the first output valve **214** into an aorta of a heart patient and concurrently the oxygen-depleted blood in the second tube **206** partially flows out through the second output valve **208** for blood oxygenation. Then, the oxygenated blood flows into the first tube **204** to wait for the squeezing of the alternating member **208**.

The advantages of the cardiopulmonary life support system according to the present invention are numerous. First, the gently alternating reciprocal movement of the alternating member squeezes the first and second tubes sequentially, alternately, gently and efficiently for blood pumping operation so that the oxygenator becomes less pressurized by the repeated blood pumping, thereby substantially decreasing incurrence of blood clotting (thrombosis) and dissolution or destruction of red blood cells (hemolysis), which are known as common side effects to most patients receiving assistance of conventional artificial hearts.

Second, the first and second tubes are formed of a flexible, resilient material and the solid alternating member is operatively provided between the first and second tubes in such a simplified, stabilized construction that the expected life span of the life support system is substantially extended without system replacement.

Third, the alternating member and the first and second tubes are efficiently accommodated within the housing to alternately enable each blood pumping operation for the first and second tube in such a limited space that a significant system size decrease is realized, for example, from a conventional refrigerator size to a palm size in an implantation version of the present invention or to a portable size in an extracorporeal assistance version of the present invention.

Fourth, the gentle, pulsatile blood pumping operation accomplished within the housing in systematic combination of the flexible blood tubes and the gently alternating solid member generates safe and steady blood pulses substantially similar to those of a natural heart, thereby improving product reliability.

Fifth, the artificial blood pumping system adapting the alternately tube-squeezing mechanism requires less elements and further simplifies the overall structure for the blood pumping operation, thereby substantially decreasing production cost, whereby a surgical implantation of the life support system may be realized, for example, within about one and half times the medical bill charged for a large surgical heart operation.

Although the invention has been described in considerable detail with reference to certain preferred versions thereof, other versions are possible by converting the aforementioned construction. Therefore, the scope of the invention shall not be limited by the specification specified above but by the appended claims.

## Claims

1. A cardiopulmonary life support system comprising:
a) A housing (76) defined by a top side (98), a bottom (100), a rear side, and an inner periphery (102);
b) first and second tubes (94, 96) adjacent to each other in the housing (76), wherein the first and second tubes (94, 96) each have an input port (94a, 96a) and an output port (94b, 96b);
c) an alternating member (104) attached to the housing (76) and disposed between the first and second tubes (94, 96), wherein the alternating member (104) alternately squeezes the first and second tubes (94, 96); and
d) a valve (106, 108, 110, 112) formed in said each input and output port to prevent a reverse stream in the first and second tubes (94, 96) **characterised in that** it further comprises:
e) a solid tube support (114, 226) fitted between said each tube and the inner periphery (102) of the housing (76); and
f) a shaft (116) substantially parallel to the tubes, wherein the shaft (116) has a top portion (118) rotatably attached to the top side (98), a mid portion (120) fixedly attached (124) to the alternating member (104), and a bottom portion (122) rotatably passing through the bottom side (100) of the housing (76), whereby an angular reciprocal rotation of the shaft (116) enables the alternating member (104) to alternately squeeze the first and second tubes (94, 96).

2. The life support system of Claim 1 further comprising:
a) a motor (126) adjacent to the housing (76);
b) a decelerator (128) connected to the motor (126); and
c) a first gear (130) having a gear base connected to the decelerator (128), wherein the first gear (130) is rotatably connected to the shaft (116).

3. The life support system of Claim 2 further comprising a second gear (132) having a gear base attached to the bottom portion (122) of the shaft (116), wherein the second gear (132) is rotatably engaged to the first gear (130).

4. The life support system of Claim 3 wherein a male spline (144) is fixed to the bottom portion (122) of the shaft (116) by a base thereof, and a female spline (146) is fixed to the second gear base by a base thereof, wherein the male spline (144) is detachable engaged to the female spline (146).

5. The life support system of Claim 1 wherein a support plate extends from the mid portion (120) of the shaft (116) fixedly to the alternating member (104) to stabilize the angular reciprocal rotation of the alternating member (104).

6. The life support system of Claim 1 wherein the first and second tubes (94, 96) are linearly aligned substantially parallel with each other.

7. The life support system of Claim 1 wherein the first and second tubes (94, 96) are each elastically, substantially restored to its original shape after being squeezed by the alternating member (104).

8. The life support system of Claim 1 wherein said each valve (106, 108, 110, 112) is a one-way valve to allow a single directional stream therethrough.

9. The life support system of Claim 1 wherein an initial squeezing of the alternating member (104) on the first tube (94) enables a blood to partially pump out from the first tube (94) through the first tube output port, wherein a subsequent squeezing of the alternating member (104) on the second tube (96) enables the blood to partially pump out form the second tube (96) through the second tube output port while a restoration of the first tube (94) to its original shape enables the first tube (94) to suck in as much as pumped out therefrom through the first input port valve (106), wherein a further subsequent squeezing of the alternating member (104) on the first tube (94) enables the blood to partially pump out from the first tube (94) though the first tube output port while a subsequent restoration of the second tube (96) to its original shape enables the second tube (96) to suck in as much as pumped out therefrom through the second input port valve (110)

10. The life support system of Claim 1 wherein the output port of the first tube (94) passes through the top side (98) and the output port of the second tube (96) passes through the bottom side (100) of the housing (76).

11. The life support system of any of claims 1 to 10 further comprising:
a) an oxygenator connected to the output port of the first tube (94) and the input port of the second tube (96) to convert an oxygen-depleted blood to an oxygen-rich blood.

12. The life support system of Claim 11 further comprising first and second blood storages, wherein the first blood storage is formed between the oxygenator and the input port of the first tube (94) to temporarily store therein the oxygen-rich blood oxygenated in the oxygenator, wherein the second blood storage is connected to the output port of the second tube (96) to temporarily store therein the oxygen-depleted blood.

13. The life support system of Claim 12 wherein an initial squeezing of the alternating member (104) on the first tube (94) enables the oxygen-rich blood to partially pump out from the first tube (94) through the first tube output port, wherein a subsequent squeezing of the alternating member (104) on the second tube (96) enables the oxygen-depleted blood to partially pump out from the second tube (96) through the second output port while a restoration of the first tube (94) to its original shape enables the first tube (94) to suck in as much as pumped out therefrom through the first input port valve (106), wherein a further subsequent squeezing of the alternating member (104) on the first tube (94) enables the oxygen-rich blood to partially pump out from the first tube (94) through the first output port while a subsequent restoration of the second tube (96) to its original shaft enables the second tube (96) to suck in as much as pumped out therefrom through the second input port valve (110).

14. The life support system of Claim 11 further comprising:
a) a motor (126) adjacent to the housing (76);
b) a decelerator (128) connected to the motor (126); and
c) a first gear (130) having a gear base connected to the decelerator (128), wherein the first gear (130) is rotatably connected to the shaft (116).

15. The life support system of Claim 14 further comprising a second gear (132) having a gear base attached to the bottom portion (122) of the shaft (116), wherein the second gear (132) is rotatably engaged to the first gear (130).

16. The life support system of Claim 15 wherein a male spline (144) is fixed to the bottom portion (122) of the shaft (116) by a base thereof, and a female spline (146) is fixed to the second gear base by a base thereof, wherein the male spline (144) is detachably engaged to the female spline (146).

17. The life support system of Claim 16 wherein a support plate extends from the mid portion (120) of the shaft (116) fixedly to the alternating member (104) to stabilize the angular reciprocal rotation of the alternating member (104).

18. The life support system of Claim 17 wherein the first and second tubes (94, 96) are linearly aligned substantially parallel with each other.

19. The life support system of Claim 18 wherein the first and second tubes (94, 96) are each elastically restored to its original shape after being squeezed by the alternating member (104).

20. The life support system of Claim 19 wherein said each valve (106, 108 110, 112) is a one-way valve to allow a single directional stream therethrough.

21. The life support system of Claim 20 wherein the output port of the first tube (94) passes through the top side (98) and the output port of the second tube (96) passes through the bottom side (100) of the housing (76).

22. The life support system of Claim 11 wherein the first tube output port is to be connected to an aorta of a mammal body and the second tube input port is to be connected to a main vein of the mammal body, whereby the oxygen-depleted blood from the main vein is oxygenated and regularly pumped out into the aorta.

## Patentansprüche

1. Herz-Lungen-Lebenserhaltungssystem, das aufweist:
a) ein Gehäuse (76), das durch eine Oberseite (98), eine Unterseite (100), eine Rückseite und eine innere Begrenzung (102) definiert wird;
b) einander benachbarte erste und zweite Schläuche (94, 96) in dem Gehäuse (76), wobei die ersten und zweiten Schläuche (94, 96) jeweils eine Eintrittsöffnung (94a, 96a) und eine Austrittsöffnung (94b, 96b) aufweisen;
c) ein alternierendes Element (104), das an dem Gehäuse (76) angebracht und zwischen den ersten und zweiten Schläuchen (94, 96) angeordnet ist, wobei das alternierende Element (104) abwechselnd die ersten und zweiten Schläuche (94, 96) zusammendrückt; und
d) ein in jeder Eintritts- und Austrittsöffnung ausgebildetes Ventil (106, 108, 110, 112), um eine Rückströmung in den ersten und zweiten Schläuchen (94, 96) zu verhindern;
**dadurch gekennzeichnet, daß** das Lebenserhaltungssystem ferner aufweist:
e) eine massive Schlauchauflage (114, 226), die zwischen jedem Schlauch und der inneren Begrenzung (102) des Gehäuses (76) eingepaßt ist; und
f) eine im wesentlichen zu den Schläuchen parallele Welle (116), wobei die Welle (116) einen drehbar an der Oberseite (98) angebrachten oberen Abschnitt (118), einen fest an dem alternierenden Element (104) angebrachten (124) mittleren Abschnitt (120) und einen drehbar durch die Unterseite (100) des Gehäuses (76) hindurchgehenden unteren Abschnitt (122) aufweist, wodurch eine hin- und hergehende Winkeldrehung der Welle (116) das alternierende Element in die Lage versetzt, die ersten und zweiten Schläuche (94, 96) abwechselnd zusammenzudrücken.

2. Lebenserhaltungssystem nach Anspruch 1, das ferner aufweist:
a) einen an das Gehäuse (76) angrenzenden Motor (126);
b) ein mit dem Motor (126) verbundenes Untersetzungsgetriebe (128); und
c) ein erstes Zahnrad (130), das einen mit dem Untersetzungsgetriebe (128) verbundenen Zahnradfuß aufweist, wobei das erste Zahnrad (130) drehbar mit der Welle (116) verbunden ist.

3. Lebenserhaltungssystem nach Anspruch 2, das ferner ein zweites Zahnrad (132) aufweist, das einen mit dem unteren Abschnitt (122) der Welle (116) verbundenen Zahnradfuß aufweist, wobei das zweite Zahnrad (132) drehbar mit dem ersten Zahnrad (130) im Eingriff ist.

4. Lebenserhaltungssystem nach Anspruch 3, wobei an dem unteren Abschnitt (122) der Welle (116) ein Keil (144) durch seinen Fuß befestigt ist und an dem Fuß des zweiten Zahnrads ein Keilnut (146) durch ihren Fuß befestigt ist, wobei der Keil (144) lösbar mit der Keilnut (146) im Eingriff ist.

5. Lebenserhaltungssystem nach Anspruch 1, wobei sich von dem mittleren Abschnitt (120) der Welle (116) eine Trägerplatte starr zu dem alternierenden Element (104) erstreckt, um die hin- und hergehende Winkeldrehung des alternierenden Elements (104) zu stabilisieren.

6. Lebenserhaltungssystem nach Anspruch 1, wobei die ersten und zweiten Schläuche (94, 96) im wesentlichen parallel zueinander linear ausgerichtet sind.

7. Lebenserhaltungssystem nach Anspruch 1, wobei die ersten und zweiten Schläuche (94, 96) jeweils nach dem Zusammendrücken durch das alternierende Element (104) im wesentlichen elastisch in ihre ursprüngliche Form zurückgeführt werden.

8. Lebenserhaltungssystem nach Anspruch 1, wobei jedes Ventil (106, 108, 110, 112) ein Einwegventil ist, um einen Fluß in einer Richtung durchzulassen.

9. Lebenserhaltungssystem nach Anspruch 1, wobei ein anfänglicher Druck des alternierenden Elements (104) auf den ersten Schlauch (94) ein teilweises Auspumpen von Blut aus dem ersten Schlauch (94) durch die erste Schlauchaustrittsöffnung ermöglicht, wobei ein anschließender Druck des alternierenden Elements (104) auf den zweiten Schlauch (96) ein teilweises Auspumpen des Bluts aus dem zweiten Schlauch (96) durch die zweite Schlauchaustrittsöffnung ermöglicht, während eine Wiederherstellung der ursprünglichen Form des ersten Schlauchs (94) ermöglicht, das der erste Schlauch (94) durch das erste Eintrittsöffnungsventil (106) so viel ansaugt, wie daraus ausgepumpt wurde, wobei ein weiterer anschließender Druck des alternierenden Elements (104) auf den ersten Schlauch (94) ein teilweises Auspumpen des Bluts aus dem ersten Schlauch (94) durch die erste Schlauchaustrittsöffnung ermöglicht, während eine anschließende Wiederherstellung der ursprünglichen Form des zweiten Schlauchs (96) ermöglicht, daß der zweite Schlauch (96) durch das zweite Eintrittsöffnungsventil (110) so viel ansaugt, wie daraus ausgepumpt wurde.

10. Lebenserhaltungssystem nach Anspruch 1, wobei die Austrittsöffnung des ersten Schlauchs (94) durch die Oberseite (98) und die Austrittsöffnung des zweiten Schlauchs (96) durch die Unterseite (100) des Gehäuses (76) geht.

11. Lebenserhaltungssystem nach einem der Ansprüche 1 bis 10, das ferner aufweist:
a) einen mit der Austrittsöffnung des ersten Schlauchs (94) und der Eintrittsöffnung des zweiten Schlauchs (96) verbundenen Oxygenator zur Umwandlung eines sauerstoffabgereicherten Bluts in sauerstoffreiches Blut.

12. Lebenserhaltungssystem nach Anspruch 11, das ferner erste und zweite Blutspeicher aufweist, wobei der erste Blutspeicher zwischen dem Oxygenator und der Eintrittsöffnung des ersten Schlauchs (94) ausgebildet ist, um darin vorübergehend das in dem Oxygenator oxygenierte sauerstoffreiche Blut zu speichern, wobei der zweite Blutspeicher mit der Austrittsöffnung des zweiten Schlauchs (96) verbunden ist, um darin vorübergehend das sauerstoffabgereicherte Blut zu speichern.

13. Lebenserhaltungssystem nach Anspruch 12, wobei ein anfänglicher Druck des alternierenden Elements (104) auf den ersten Schlauch (94) ein teilweises Auspumpen des sauerstoffreichen Bluts aus dem ersten Schlauch (94) durch die erste Schlauchaustrittsöffnung ermöglicht, wobei ein anschließender Druck des alternierenden Elements (104) auf den zweiten Schlauch (96) ein teilweises Auspumpen des sauerstoffabgereicherten Bluts aus dem zweiten Schlauch (96) durch die zweite Austrittsöffnung ermöglicht, während eine Wiederherstellung der ursprünglichen Form des ersten Schlauchs (94) ermöglicht, daß der erste Schlauch (94) durch das erste Eintrittsöffnungsventil (106) so viel ansaugt, wie daraus ausgepumpt wurde, wobei ein weiterer anschließender Druck des alternierenden Elements (104) auf den ersten Schlauch (94) das teilweise Auspumpen des sauerstoffreichen Bluts aus dem ersten Schlauch (94) durch die erste Austrittsöffnung ermöglicht, während eine anschließende Wiederherstellung der ursprünglichen Form des zweiten Schlauchs (96) ermöglicht, daß der zweite Schlauch (96) durch das zweite Eintrittsöffnungsventil (110) so viel ansaugt, wie daraus ausgepumpt wurde.

14. Lebenserhaltungssystem nach Anspruch 11, das ferner aufweist:
a) einen an das Gehäuse (76) angrenzenden Motor (126);
b) ein mit dem Motor (126) verbundenes Untersetzungsgetriebe (128); und
c) ein erstes Zahnrad (130), das einen mit dem Untersetzungsgetriebe (128) verbundenen Zahnradfuß aufweist, wobei das erste Zahnrad (130) drehbar mit der Welle (116) verbunden ist.

15. Lebenserhaltungssystem nach Anspruch 14, das ferner ein zweites Zahnrad (132) aufweist, das einen mit dem unteren Abschnitt (122) der Welle (116) verbundenen Zahnradfuß aufweist, wobei das zweite Zahnrad (132) drehbar mit dem ersten Zahnrad (130) im Eingriff ist.

16. Lebenserhaltungssystem nach Anspruch 15, wobei an dem unteren Abschnitt (122) der Welle (116) ein Keil (144) durch seinen Fuß befestigt ist und an dem Fuß des zweiten Zahnrads ein Keilnut (146) durch ihren Fuß befestigt ist, wobei der Keil (144) lösbar mit der Keilnut (146) im Eingriff ist.

17. Lebenserhaltungssystem nach Anspruch 16, wobei sich von dem mittleren Abschnitt (120) der Welle (116) eine Trägerplatte starr zu dem alternierenden Element (104) erstreckt, um die hin- und hergehende Winkeldrehung des alternierenden Elements (104) zu stabilisieren.

18. Lebenserhaltungssystem nach Anspruch 17, wobei die ersten und zweiten Schläuche (94, 96) im wesentlichen parallel zueinander linear ausgerichtet sind.

19. Lebenserhaltungssystem nach Anspruch 18, wobei die ersten und zweiten Schläuche (94, 96) jeweils nach dem Zusammendrücken durch das alternierende Element (104) elastisch in ihre ursprüngliche Form zurückgeführt werden.

20. Lebenserhaltungssystem nach Anspruch 19, wobei jedes Ventil (106, 108, 110, 112) ein Einwegventil ist, um einen Fluß in einer Richtung durchzulassen.

21. Lebenserhaltungssystem nach Anspruch 20, wobei die Austrittsöffnung des ersten Schlauchs (94) durch die Oberseite (98) und die Austrittsöffnung des zweiten Schlauchs (96) durch die Unterseite (100) des Gehäuses (76) geht.

22. Lebenserhaltungssystem nach Anspruch 11, wobei die Austrittsöffnung des ersten Schlauchs mit einer Aorta eines Säugetierkörpers und die Eintrittsöffnung des zweiten Schlauchs mit einer Hauptvene des Säugetierkörpers zu verbinden ist, wodurch das sauerstoffabgereicherte Blut von der Hauptvene mit Sauerstoff angereichert und regelmäßig in die Aorta ausgepumpt wird.

## Revendications

1. Système de maintien des fonctions vitales cardio-pulmonaires, comprenant:
a) un logement (76) défini par un côté du dessus (98), un fond (100), un côté arrière, et une périphérie interne (102);
b) des premier et second tubes (94, 96) adjacents entre eux dans le logement (76), dans lequel les premier et second tubes (94, 96) ont chacun une ouverture d'arrivée (94a, 96a) et une ouverture de sortie (94b, 96b);
c) un élément à mouvement alternatif (104) attaché au logement (76) et disposé entre les premier et second tubes (94, 96), l'élément à mouvement alternatif (104) comprimant en alternance les premier et second tubes (94, 96); et
d) une valve (106, 108, 110, 112) formée dans chaque ouverture d'arrivée et de sortie pour prévenir une inversion d'écoulement dans les premier et second tubes (94, 96),
**caractérisé en ce qu'**il comprend en outre:
e) un support tubulaire plein (114, 226) ajusté entre chacun desdits tubes et la périphérie interne (102) du logement (76); et
f) un arbre (116) essentiellement parallèle aux tubes, l'arbre (116) ayant une partie du dessus (118) attachée de façon rotative au côté du dessus (98), une partie médiane (120) attachée de façon fixe (124) à l'élément à mouvement alternatif (104), et une partie fond (122) passant de façon rotative à travers le côté fond (100) du logement (76), de sorte qu'une rotation réciproque angulaire de l'arbre (116) permet à l'élément à mouvement alternatif (104) de comprimer en alternance les premier et second tubes (94, 96).

2. Système de maintien des fonctions vitales selon la revendication 1, comprenant en outre:
a) un moteur (126) adjacent au logement (76);
b) un décélérateur (128) raccordé au moteur (126); et
c) un premier engrenage (130) ayant une base d'engrenage raccordée au décélérateur (128), dans lequel le premier engrenage (130) est raccordé de façon rotative à l'arbre (116).

3. Système de maintien des fonctions vitales selon la revendication 2, comprenant en outre un second engrenage (132) ayant une base d'engrenage attachée à la partie fond (122) de l'arbre (116), dans lequel le second engrenage (132) est engagé de façon rotative avec le premier engrenage (130).

4. Système de maintien des fonctions vitales selon la revendication 3, dans lequel une cannelure mâle (144) est fixée à la partie fond (122) de l'arbre (116) par une base de celle-ci, et une cannelure femelle (146) est fixée à la base du second engrenage par une base de celle-ci, dans lequel la cannelure mâle (144) est engagée de façon détachable avec la cannelure femelle (146).

5. Système de maintien des fonctions vitales selon la revendication 1, dans lequel une plaque de support s'étend depuis la partie médiane (120) de l'arbre (116) de façon fixe à l'élément à mouvement alternatif (104) pour stabiliser la rotation réciproque angulaire de l'élément à mouvement alternatif (104).

6. Système de maintien des fonctions vitales selon la revendication 1, dans lequel les premier et second tubes (94, 96) sont alignés linéairement essentiellement parallèles l'un avec l'autre.

7. Système de maintien des fonctions vitales selon la revendication 1, dans lequel les premier et second tubes (94, 96) reprennent chacun élastiquement essentiellement leur forme d'origine après avoir été comprimés par l'élément à mouvement alternatif (104).

8. Système de maintien des fonctions vitales selon la revendication 1, dans lequel chacune desdites valves (106, 108, 110, 112) est une valve antireflux pour permettre un écoulement dans un sens unique à travers celles-ci.

9. Système de maintien des fonctions vitales selon la revendication 1, dans lequel une compression initiale de l'élément à mouvement alternatif (104) sur le premier tube (94) permet au sang d'être partiellement pompé hors du premier tube (94) à travers l'ouverture de sortie du premier tube, dans lequel une compression subséquente de l'élément à mouvement alternatif (104) sur le second tube (96) permet au sang d'être partiellement pompé hors du second tube (96) à travers l'ouverture de sortie du second tube pendant qu'un retour du premier tube (94) à sa forme d'origine permet au premier tube (94) d'aspirer autant que ce qui en a été enlevé par pompage à travers la valve de la première ouverture d'arrivée (106), dans lequel une autre compression subséquente de l'élément à mouvement alternatif (104) sur le premier tube (94) permet au sang d'être partiellement pompé hors du premier tube (94) à travers l'ouverture de sortie du premier tube pendant qu'un retour subséquent du second tube (96) à sa forme d'origine permet au second tube (96) d'aspirer autant que ce qui en a été enlevé par pompage à travers la valve de la deuxième ouverture d'arrivée (110).

10. Système de maintien des fonctions vitales selon la revendication 1, dans lequel l'ouverture de sortie du premier tube (94) passe à travers le côté du dessus (98) et l'ouverture de sortie du second tube (96) passe à travers le côté fond (100) du logement (76).

11. Système de maintien des fonctions vitales selon l'une quelconque des revendications 1 à 10, comprenant en outre:
a) un oxygénateur raccordé à l'ouverture de sortie du premier tube (94) et à l'ouverture d'arrivée du second tube (96) pour convertir un sang épuisé en oxygène en un sang riche en oxygène.

12. Système de maintien des fonctions vitales selon la revendication 11, comprenant en outre des premier et second stockages de sang, dans lequel le premier stockage de sang est formé entre l'oxygénateur et l'ouverture d'arrivée du premier tube (94) pour stocker temporairement dans celui-ci le sang riche en oxygène oxygéné dans l'oxygénateur, dans lequel le second stockage de sang est raccordé à l'ouverture de sortie du second tube (96) pour stocker temporairement dans celui-ci le sang épuisé en oxygène.

13. Système de maintien des fonctions vitales selon la revendication 12, dans lequel une compression initiale de l'élément à mouvement alternatif (104) sur le premier tube (94) permet au sang riche en oxygène d'être partiellement pompé hors du premier tube (94) à travers l'ouverture de sortie du premier tube, dans lequel une compression subséquente de l'élément à mouvement alternatif (104) sur le second tube (96) permet au sang épuisé en oxygène d'être partiellement pompé hors du second tube (96) à travers la seconde ouverture de sortie pendant qu'un retour du premier tube (94) à sa forme d'origine permet au premier tube (94) d'aspirer autant que ce qui en a été enlevé par pompage à travers la valve de la première ouverture d'arrivée (106), dans lequel une autre compression subséquente de l'élément à mouvement alternatif (104) sur le premier tube (94) permet au sang riche en oxygène d'être partiellement pompé hors du premier tube (94) à travers la première ouverture de sortie pendant qu'un retour subséquent du second tube (96) à sa forme d'origine permet au second tube (96) d'aspirer autant que ce qui en a été enlevé par pompage à travers la valve de la deuxième ouverture d'arrivée (110).

14. Système de maintien des fonctions vitales selon la revendication 11, comprenant en outre:
a) un moteur (126) adjacent au logement (76);
b) un décélérateur (128) raccordé au moteur (126); et
c) un premier engrenage (130) ayant une base d'engrenage raccordée au décélérateur (128), le premier engrenage (130) étant raccordé de façon rotative à l'arbre (116).

15. Système de maintien des fonctions vitales selon la revendication 14, comprenant en outre un second engrenage (132) ayant une base d'engrenage attachée à la partie fond (122) de l'arbre (116), dans lequel le second engrenage (132) est engagé de façon rotative avec le premier engrenage (130).

16. Système de maintien des fonctions vitales selon la revendication 15, dans lequel une cannelure mâle (144) est fixée à la partie fond (122) de l'arbre (116) par une base de celle-ci, et une cannelure femelle (146) est fixée à la base du second engrenage par une base de celle-ci, dans lequel la cannelure mâle (144) est engagée de façon détachable avec la cannelure femelle (146).

17. Système de maintien des fonctions vitales selon la revendication 16, dans lequel une plaque de support s'étend à partir de la partie médiane (120) de l'arbre (116) de façon fixe à l'élément à mouvement alternatif (104) pour stabiliser la rotation réciproque angulaire de l'élément à mouvement alternatif (104).

18. Système de maintien des fonctions vitales selon la revendication 17, dans lequel les premier et second tubes (94, 96) sont alignés linéairement essentiellement parallèles l'un avec l'autre.

19. Système de maintien des fonctions vitales selon la revendication 18, dans lequel les premier et second tubes (94, 96) reprennent chacun élastiquement leur forme d'origine après avoir été comprimés par l'élément à mouvement alternatif (104).

20. Système de maintien des fonctions vitales selon la revendication 19, dans lequel chacune desdites valves (106, 108, 110, 112) est une valve antireflux pour permettre un écoulement dans un sens unique à travers celles-ci.

21. Système de maintien des fonctions vitales selon la revendication 20, dans lequel l'ouverture de sortie du premier tube (94) passe à travers le côté du dessus (98) et l'ouverture de sortie du second tube (96) passe à travers le côté fond (100) du logement (76).

22. Système de maintien des fonctions vitales selon la revendication 11, dans lequel l'ouverture de sortie du premier tube doit être raccordée à une aorte d'un corps mammifère, et l'ouverture d'arrivée du second tube doit être raccordée à une veine principale du corps mammifère, de sorte que le sang épuisé en oxygène de la veine principale est oxygéné et régulièrement pompé jusque dans l'aorte.
